Europäisches Patentamt

European Patent Office

Office européen des brevets

(11) Publication number: **0 299 527 B1**

# EUROPEAN PATENT SPECIFICATION

(45) Date of publication of patent specification: **15.04.92**

(51) Int. Cl.⁵: **A61K 31/71**, A61K 47/12, A61K 47/08

(21) Application number: **88111433.4**

(22) Date of filing: **15.07.88**

The file contains technical information submitted after the application was filed and not included in this specification

(54) **Doxorubicin hydrochloride aqueous solutions.**

(30) Priority: **16.07.87 US 74190**

(43) Date of publication of application:
**18.01.89 Bulletin 89/03**

(45) Publication of the grant of the patent:
**15.04.92 Bulletin 92/16**

(84) Designated Contracting States:
**AT BE CH DE ES FR GB GR IT LI LU NL SE**

(56) References cited:
**EP-A- 0 010 437
EP-A- 0 219 784
GB-A- 2 178 311**

(73) Proprietor: **Bristol-Myers Squibb Company
345 Park Avenue
New York, N.Y. 10154(US)**

(72) Inventor: **Kaplan, Murray Arthur
1026 Glencove Road
Syracuse, N.Y. 13206(US)**
Inventor: **Perrone, Robert Kevin
7353 Tomwood Drive
Liverpool, N.Y. 13090(US)**
Inventor: **Bogardus, Joseph Ballard, Dr.
8239 Penstock Way
Syracuse, N.Y. 13104(US)**

(74) Representative: **Kinzebach, Werner, Dr.
Patentanwälte Reitstötter, Kinzebach und
Partner Sternwartstrasse 4 Postfach 86 06 49
W-8000 München 86(DE)**

## Description

The present invention relates to stable, ready-to-use aqueous solutions of doxorubicin hydrochloride suitable for parenteral administration.

Doxorubicin is an antineoplastic antibiotic which is isolated from Streptomyces peucetius var. caesius. It can also be synthesized chemically from daunorubicin.

The commercial lyophilized dosage form of doxorubicin (marketed by Adria Laboratories as Adriamycin®) is supplied as the hydrochloride salt in 10, 20 and 50 mg vials in combination with lactose at 50, 100 and 250 mg, respectively. Vials are reconstituted with Sodium Chloride Injection, USP (0.9%), or Sterile Water for Injection, USP, to give a final concentration of 2 mg/ml of doxorubicin HCl.

It would be desirable to have a ready-to-use aqueous solution dosage form of doxorubicin HCl which would not require reconstitution prior to use. Improper reconstitution of a lyophilized product sometimes results in the formation of air-borne droplets ("blow-back") which, in the case of a potent antitumor agent such as doxorubicin HCl, may be a health hazard to the personnel making up the solution for injection. In addition, there is always a problem in reconstituting a lyophilized formulation in that an inappropriate quantity of diluent may be used or an incorrect amount of drug may be used because of a different vial size. Finally, production of the present lyophilized doxorubicin HCl dosage form is quite costly, and a ready-to-use solution would be expected to have a lower cost-of-goods.

Despite the advantages of a ready-to-use aqueous solution form of doxorubicin HCl, this drug has not heretofore been available in such form because of its inherent instability in aqueous systems. The manufacturer of Adriamycin® indicates that the reconstituted lyophilized solution is stable for only 24 hours at room temperature and 48 hours under refrigeration (4°C).

A study carried out by Hoffman, et al. and reported in Am. J. Hosp. Pharm. 36: 1536-1538, 1979 indicates that reconstituted solutions of doxorubicin HCl are stable (≤10% potency loss) for up to six months when refrigerated at 4°C. Ketchum, et al. in Am. J. Intrav. Ther. Clin. Nutri. 8: 15-18, 1981, state that a reconstituted solution of doxorubicin HCl in 0.9% NaCl solution remains stables at room temperature or 5°C for seven days. Despite such indications that reconstituted doxorubicin HCl may be sufficiently stable for up to six months at 4°C, this is still undesirable for a commercial ready-to-use dosage form.

Janssen, et al. in Int. J. Pharmaceutics 23: 1-11, 1985, report that doxorubicin HCl appears to have its maximum stability at pH 4 (Tris or phosphate buffers using HCl to adjust pH). Poochikian, et al. in Am. J. Hosp. Pharm. 38: 483-486, 1981, note that the stability of doxorubicin HCl is pH dependent and that the optimum stability is achieved when the reconstitution solvent is 5% Dextrose Injection, USP, having a pH~4.5.

U. K. Patent Application 2,178,311A discloses ready-to-use aqueous solutions of doxorubicin HCl. However, there is no disclosure of the use of an antioxidant selected from α-tocopherol, sodium formaldehyde bisulfite and tert-butylhydroquinone which we have found imparts unexpectedly improved stability at elevated temperatures (e.g. 37°C) compared to the solutions of U. K. 2,178,311A.

It is an object of the present invention to provide a ready-to-use aqueous solution of doxorubicin HCl which is stable (≤10% potency loss) for at least two years at 4°C and at least one month at 37°C.

## SUMMARY OF THE INVENTION

The present invention provides a stable, sterile, ready-to-use solution in a sealed container suitable for parenteral administration containing doxorubicin hydrochloride, an amount of citric acid necessary to provide a solution pH in the range of 3-4, 0.1-2 mg/ml of an antioxidant selected from sodium formaldehyde bisulfite, α-tocopherol and tert-butylhydroquinone, and water.

## DETAILED DESCRIPTION

This invention relates to stable, aqueous, ready-to-use solutions of doxorubicin HCl suitable for parenteral administration. Although concentrations of from 2-20 mg/ml doxorubicin HCl may be employed in the solutions, it is preferred to use from 2-5 mg/ml of doxorubicin HCl and, most preferably 2 mg/ml.

One critical component of the solutions in pH with a pH range of 3-4 being necessary for acceptable long-term stability. While a number of acids may be used to achieve this pH range, we have found that optimum stability is achieved with citric acid since this acid acts as a metal chelation agent as well as providing pH control. An amount of citric acid is used which will provide a solution pH in the range of 3-4. Generally a concentration of citric acid of 0.2 mg/ml will provide this pH control.

It is also necessary for optimum stability of the aqueous solutions, especially at elevated temperatures which may be encountered at some time during the life of the product, e.g. during shipping, to utilize a particular antioxidant. We have tested a number of antioxidants including tert-butylhydroquinone (TBHQ), sodium formaldehyde bisulfite, butylated hydroxy anisole (BHA), butylated hydroxy toluene (BHT), sodium bisulfite, $\alpha$-tocopherol and propyl gallate. Only tert-butylhydroquinone, $\alpha$-tocopherol and sodium formaldehyde bisulfite resulted in the desired stability at elevated temperatures as well as at 4°C. Generally an amount of tert-butylhydroquinone, $\alpha$-tocopherol or sodium formaldehyde bisulfite is employed in the range of 0.1-2 mg/ml. The preferred concentration of antioxidant is 0.5-1 mg/ml with 1 mg/ml being most preferred.

In our studies the use of buffers such as acetate and citrate buffers had a deleterious effect on stability of the solutions and the presence of surfactants such as Tween®-80 did not improve stability.

Despite reports of potency losses of doxorubicin HCl solutions via photolytic degradation and adsorption to container walls, we have not noted any significant loss of potency due to container adsorption or sensitivity to light with our solutions. Glass vials may be used as the container or, alternatively, the solution may be filled into hypodermic syringes of pre-determined volume to provide a ready-to-use solution which is also ready to inject.

We have also found that deaeration of the water (e.g. by boiling and/or vacuum prior to formulation) and nitrogen purging of the formulation filled vials before they are sealed contributes to optimum stability and is preferred. Nitrogen purging is especially preferred for our solutions.

As an example of a stable ready-to-use aqueous solution of doxorubicin HCl, 2.0 mg/ml doxorubicin HCl, 0.2 mg/ml citric acid (anhydrous), 1.0 mg/ml of TBHQ and sterile oxygen-free water are added to a Type I Flint vial. The vial is purged with nitrogen and then sealed with a suitable Teflon coated stopper. Alternatively, 1 mg/ml of sodium formaldehyde bisulfite or $\alpha$-tocopherol may be used as the antioxidant. Vials containing larger concentrations of doxorubicin HCl are prepared in a similar manner.

Elevated temperature stability studies indicate that our ready-to-use aqueous solutions have a shelf-life (≤10% potency loss) of at least two years at 4°C, at least one month at 37°C and at least six months at 25°C.

The ready-to-use solutions of the present invention are used for the treatment of cancer in the same manner as the present reconstituted lyophilized dosage form.

## Claims

**Claims for the following Contracting States :**
**AT, BE, CH, DE, FR, GB, IT, LI, LU, NL, SE**

1. A pharmaceutical formulation of a stable, sterile, ready-to-use aqueous solution in a sealed container suitable for parenteral administration containing 2-5 mg/ml of doxorubicin hydrochloride, an amount of citric acid necessary to provide a solution pH in the range of 3-4, 0.1-2 mg/ml of an antioxidant selected from $\alpha$-tocopherol, sodium formaldehyde bisulfite and tert-butylhydroquinone, and water.

2. A formulation according to Claim 1 wherein deaerated water is employed and the solution is subjected to nitrogen purging before the container is sealed.

3. A formulation according to Claim 1 or Claim 2 wherein the antioxidant is sodium formaldehyde bisulfite.

4. A formulation according to Claim 1 or Claim 2 wherein the antioxidant is tert-butylhydroquinone.

5. A formulation according to Claim 1 or Claim 2 wherein the antioxidant is $\alpha$-tocopherol.

6. A pharmaceutical formulation according to Claim 1 containing per ml of solution, 2 mg doxorubicin hydrochloride, 0.2 mg citric acid, 1 mg tert-butylhydroquinone, and deaerated water qs, said solution being subjected to nitrogen purging prior to sealing of the container.

7. A pharmaceutical formulation according to Claim 1 containing per ml of solution 2 mg doxorubicin hydrochloride, 0.2 mg citric acid, 1 mg sodium formaldehyde bisulfite, and deaerated water qs, said solution being subjected to nitrogen purging prior to sealing of the container.

8. A pharmaceutical formulation according to Claim 1 containing per ml of solution 2 mg doxorubicin hydrochloride, 0.2 mg citric acid, 1 mg $\alpha$-tocopherol, and deaerated water qs, said solution being subjected to nitrogen purging prior to sealing of the container.

**Claims for the following Contracting States :**
**ES, GR**

1. A process for preparing a pharmaceutical formulation of a stable, sterile, ready-to-use aqueous solution in a sealed container suitable for parenteral administration containing about 2-5 mg/ml of doxorubicin hydrochloride, an amount of citric acid necessary to provide a solution pH in the range of about 3-4, 0.1-2 mg/ml of an antioxidant selected from α-tocopherol, sodium formaldehyde bisulfite and tert-butylhydroquinone, and water.

2. A process according to Claim 1 wherein deaerated water is employed and the solution is subjected to nitrogen purging before the container is sealed.

3. A process according to Claim 1 or Claim 2 wherein the antioxidant is sodium formaldehyde bisulfite.

4. A process according to Claim 1 or Claim 2 wherein the antioxidant is tert-butylhydroquinone.

5. A process according to Claim 1 or Claim 2 wherein the antioxidant is α-tocopherol.

6. A process according to claim 1 wherein a formulation is obtained containing per ml of solution, 2 mg doxorubicin hydrochloride, 0.2 mg citric acid, 1 mg tert-butylhydroquinone, and deaerated water qs, said solution being subjected to nitrogen purging prior to sealing of the container.

7. A process according to claim 1 wherein a formulation is obtained containing per ml of solution 2 mg doxorubicin hydrochloride, 0.2 mg citric acid, 1 mg sodium formaldehyde bisulfite, and deaerated water qs, said solution being subjected to nitrogen purging prior to sealing of the container.

8. A process according to claim 1 wherein a formulation is obtained containing per ml of solution about 2 mg doxorubicin hydrochloride, 0.2 mg citric acid, about 1 mg α-tocopherol, and deaerated water qs, said solution being subjected to nitrogen purging prior to sealing of the container.

**Revendications**
**Revendications pour les Etats contractants suivants : AT, BE, CH, DE, FR, GB, IT, LI, LU, NL, SE**

1. Formule pharmaceutique d'une solution aqueuse stable, stérile, prête à l'utilisation dans un conteneur scellé, appropriée à une administration parentérale, contenant 2-5 mg/ml de chlorhydrate de doxorubicine, une quantité d'acide citrique nécessaire pour produire un pH de la solution compris entre 3 et 4, 0,1-2 mg/ml d'un antioxydant choisi parmi l'α-tocophérol, le bisulfite de formaldéhyde de sodium et la tert-butylhydroquinone et de l'eau.

2. Formule selon la revendication 1 où de l'eau dégazée est employée et la solution est soumise à une purge à l'azote avant que le conteneur ne soit scellé.

3. Formule selon la revendication 1 où la revendication 2, où l'antioxydant est le bisulfite de formaldéhyde de sodium.

4. Formule selon la revendication 1 où la revendication 2, où l'antioxydant est la tert-butylhydroquinone.

5. Formule selon la revendication 1 où la revendication 2, où l'antioxydant est l'α-tocophérol.

6. Formule pharmaceutique selon la revendication 1 contenant, par ml de la solution, 2 mg de chlorhydrate de doxorubicine, 0,2 mg d'acide citrique, 1 mg de tert-butylhydroquinone et de l'eau dégazée sq, ladite solution étant soumise à une purge à l'azote avant de sceller le conteneur.

7. Formule pharmaceutique selon la revendication 1, contenant, par ml de la solution, 2 mg de chlorhydrate de doxorubicine, 0,2 mg d'acide citrique, 1 mg de bisulfite de formaldéhyde de sodium et de l'eau dégazée sq, ladite solution étant soumise à une purge à l'azote avant de sceller le conteneur.

8. Formule pharmaceutique selon la revendication 1, contenant, par ml de la solution, 2 mg de chlorhydrate de doxorubicine, 0,2 mg d'acide citrique, 1 mg d'α-tocophérol et de l'eau dégazée sq, ladite solution étant soumise à une purge à l'azote avant de sceller le conteneur.

**Revendications pour les Etats contractants suivants : ES, GR**

1. Un procédé de preparation d'une formule pharmaceutique d'une solution aqueuse stable, stérile, prête à l'utilisation dans un conteneur scellé, appropriée à une administration parentérale, contenant 2-5 mg/ml de chlorhydrate de doxorubicine, une quantité d'acide citrique nécessaire pour produire un pH de la solution compris entre 3 et 4, 0,1-2 mg/ml d'un antioxydant choisi parmi l'α-tocophérol, le bisulfite de formaldéhyde de sodium et la tert-butylhydroquinone et de l'eau.

2. Prodédé selon la revendication 1 où de l'eau dégazée est employée et la solution est soumise à une purge à l'azote avant que le conteneur ne soit scellé.

3. Procédé selon la revendication 1 où la revendication 2, où l'antioxydant est le bisulfite de formaldéhyde de sodium.

4. Procédé selon la revendication 1 où la revendication 2, où l'antioxydant est la tert-butylhydroquinone.

5. Procédé selon la revendication 1 où la revendication 2, où l'antioxydant est l'α-tocophérol.

6. Procédé selon la revendication 1, où une formule est obtenue, contenant, par ml de la solution, 2 mg de chlorhydrate de doxorubicine, 0,2 mg d'acide citrique, 1 mg de tert-butylhydroquinone et de l'eau dégazée sq, ladite solution étant soumise à une purge à l'azote avant de sceller le conteneur.

7. Procédé selon la revendication 1, où une formule est obtenue, contenant, par ml de la solution, 2 mg de chlorhydrate de doxorubicine, 0,2 mg d'acide citrique, 1 mg de bisulfite de formaldéhyde de sodium et de l'eau dégazée sq, ladite solution étant soumisè à une purge à l'azote avant de sceller le conteneur.

8. Procédé selon la revendication 1, où une formule est obtenue, contenant, par ml de la solution, 2 mg de chlorhydrate de doxorubicine, 0,2 mg d'acide citrique, 1 mg d'α-tocophérol et de l'eau dégazée sq, ladite solution étant soumise à une purge à l'azote avant de sceller le conteneur.

**Patentansprüche**
**Patentansprüche für folgende Vertragsstaaten : AT, BE, CH, DE, FR, GB, IT, LI, LU, NL, SE**

1. Pharmazeutische Formulierung aus einer stabilen, sterilen, gebrauchsfertigen, wäßrigen Lösung in einem geschlossenen, zur parenteralen Anwendung geeigneten Behälter, enthaltend 2-5 mg/ml Doxorubicinhydrochlorid, die zur Einstellung des pH-Wertes der Lösung im Bereich von 3-4 erforderliche Menge Zitronensaure, 0,1-2 mg/ml eines Antioxidationsmittels, ausgewählt unter α-Tocopherol, Natriumformaldehydbisulfit und tert-Butylhydrochinon, und Wasser.

2. Formulierung nach Anspruch 1, worin entgastes Wasser verwendet wird und die Lösung mit Stickstoff gespült wird, bevor der Behälter verschlossen wird.

3. Formulierung nach Anspruch 1 oder 2, worin das Antioxidationsmittel Natriumformaldehydbisulfit ist.

4. Formulierung nach Anspruch 1 oder 2, worin das Antioxidationsmittel tert-Butylhydrochinon ist.

5. Formulierung nach Anspruch 1 oder 2, worin das Antioxidationsmittel α-Tocopherol ist.

6. Pharmazeutische Formulierung nach Anspruch 1, die pro ml Lösung 2 mg Doxorubicin, 0,2 mg Zitronensäure, 1 mg tert-Butylhydrochinon, und entgastes Wasser qs enthält, wobei die Lösung mit Stickstoff gespült wird, bevor der Behälter verschlossen wird.

7. Pharmazeutische Formulierung nach Anspruch 1, die pro ml Lösung 2 mg Doxorubicinhydrochlorid, 0,2 mg Zitronensäure, 1 mg Natriumformaldehydbisulfit und entgastes Wasser qs enthält, wobei die Lösung mit Stickstoff gespült wird, bevor der Behälter verschlossen wird.

8. Pharmazeutische Formulierung nach Anspruch 1, die pro ml Lösung 2 mg Doxorubicinhydrochlorid, 0,2 mg Zitronensäure, 1 mg α-Tocopherol und entgastes Wasser qs enthält, wobei die Lösung mit Stickstoff gespült wird, bevor der Behälter verschlossen wird.

**Patentansprüche für folgende Vertragsstaaten : ES, GR**

1. Verfahren zur Herstellung einer pharmazeutischen Formulierung aus einer stabilen, sterilen, gebrauchsfertigen, wäßrigen Lösung in einem geschlossenen, zur parenteralen Anwendung geeigneten Behälter, enthaltend 2-5 mg/ml Doxorubicinhydrochlorid, die zur Einstellung des pH-Wertes der Lösung im Bereich von 3-4 erforderliche Menge Zitronensäure, 0,1-2 mg/ml eines Antioxidationsmittels, ausgewählt unter α-Tocopherol, Natriumformaldehydbisulfit und tert-Butylhydrochinon, und Wasser.

2. Verfahren nach Anspruch 1, worin entgastes Wasser verwendet wird und die Lösung mit Stickstoff gespült wird, bevor der Behälter verschlossen wird.

3. Verfahren nach Anspruch 1 oder 2, worin das Antioxidationsmittel Natriumformaldehydbisulfit ist.

4. Verfahren nach Anspruch 1 oder 2, worin das Antioxidationsmittel tert-Butylhydrochinon ist.

5. Verfahren nach Anspruch 1 oder 2, worin das Antioxidationsmittel α-Tocopherol ist.

6. Verfahren nach Anspruch 1, wobei man eine Formulierung erhält, die pro ml Lösung 2 mg Doxorubicin, 0,2 mg Zitronensäure, 1 mg tert-Butylhydrochinon, und entgastes Wasser qs enthält, wobei die Lösung mit Stickstoff gespült wird, bevor der Behälter verschlossen wird.

7. Verfahren nach Anspruch 1, wobei man eine Formulierung erhält, die pro ml Lösung 2 mg Doxorubicinhydrochlorid, 0,2 mg Zitronensäure, 1 mg Natriumformaldehydbisulfit und entgastes Wasser qs enthält, wobei die Lösung mit Stickstoff gespült wird, bevor der Behälter verschlossen wird.

8. Verfahren nach Anspruch 1, wobei man ein Formulierung erhält, die pro ml Lösung 2 mg Doxorubicinhydrochlorid, 0,2 mg Zitronensäure, 1 mg α-Tocopherol und entgastes Wasser qs enthält, wobei die Lösung mit Stickstoff gespült wird, bevor der Behälter verschlossen wird.